# EUROPEAN PATENT SPECIFICATION

(11) **EP 1 457 773 B1**
(45) Date of publication and mention of the grant of the patent: **03.05.2006**
(21) Application number: 02805479.9
(22) Date of filing: 19.12.2002
(51) Int. Cl.: G01N 27/416, G01N 27/30, G01N 33/49, C08G 73/06, C08G 61/12, A61B 5/00

(54) **ELECTRODE FOR ACTIVE OXYGEN SPECIES AND SENSOR USING THE ELECTRODE**
ELEKTRODE FÜR AKTIVE SAUERSTOFFSPEZIES UND DIE ELEKTRODE VERWENDENDER SENSOR
ELECTRODE POUR ESPECE CHIMIQUE D'OXYGENE ACTIF ET CAPTEUR UTILISANT L'ELECTRODE

(30) Priority: 20.12.2001 JP 2001387899
(43) Date of publication of application: 15.09.2004
(73) Proprietor: Yuasa, Makoto, Soka-shi, Saitama 340-0021 (JP); Abe, Masahiko, Noda-city, Chiba 278-0017 (JP); Takebayashi, Hitoshi, Tsukuba-shi, Ibaraki 305-0045 (JP)
(72) Inventor: YUASA, Makoto, Soka-shi, Saitama 340-0021 (JP); ABE, Masahiko, Noda-shi, Chiba 278-0017 (JP); YAMAGUCHI, Aritomo, Aoba-ku, Yokohama-shi, Kanagawa 227-0034 (JP); SHIOZAWA, Asako, Saitama-shi, Saitama 338-0006 (JP); ISHIKAWA, Masuhide, Kazo-shi, Saitama 347-0068 (JP); EGUCHI, Katsuya, Higashikurume-shi, Tokyo 203-0054 (JP); KIDO, Shigeru, c/o Boron Japan Co., Ltd., Shibuya-ku, Tokyo 150-0002 (JP)
(74) Representative: Browne, Robin Forsythe
(86) International application number: PCT/JP2002/013287
(87) International publication number: WO 2003/054536

(56) References cited:
- JIAN CHEN: 'Superoxide sensor based on hemin modified electrode' SENSORS AND ACTUATORS B vol. 70, no. 113, 2000, pages 115 - 120, XP004224589
- TOMOHISA KASANUKI: 'Hydrogen peroxide sensor based on carbon paste electrode containing a metal porphyrin complex' CHEMICAL SENSORS vol. 17, no. SUPPL.B, 04 December 2001, pages 427 - 429, XP002967702
- MITSUYASU KAWAKAMI: 'Environmental pollutant sensor based on porphyrin cobalt(11)-modified gold electrode, reports of electronics research laboratory' FUKUOKA INSTITUTE OF TECHNOLOGY vol. 18, 31 October 2001, pages 87 - 92, XP002967703
- BRUNET A.: 'Advantages and limits of the electrochemical method using nafion and Ni-porphyrin-coated microelectrode to monitor NO release from cultured vascular cells' ANALUSIS vol. 28, no. 6, 2000, pages 469 - 474, XP002967704
- ALLEN BARRY W.: 'Electrode materials for nitric oxide detection' NITRIC OXIDE vol. 4, no. 1, 2000, pages 75 - 84, XP002967705
- MAKOTO YUASA: 'Sanso kangen denkyoku shokubai to shite no daikanjo kinzoku sakutai' HYOMEN vol. 36, no. 3, 1998, pages 157 - 166, XP002967706
- MAKOTO YUASU: 'Konzoku porphyrin-rui shushoku denkyokukei no sanso kangen toksei' HYOMEN vol. 32, no. 10, 1994, pages 680 - 692, XP002967707
- INES BATINIC HABERLE: 'Relationship among redox potentials, proton dissociation constants of pyrrolic nitrogens and in vivo and in vitro superoxide dismutating activities of manganese (111) and iron(11) water-soluble porphyrins' INORGANIC CHEMISTRY vol. 38, no. 182, 1999, pages 4011 - 4022, XP001037112

## Description

### TECHNICAL FIELD

The present invention relates to an electrode for active oxygen species in the living body such as superoxide anion radicals (O₂⁻·) and to a sensor for measuring the concentration of active oxygen species using the electrode. More specifically, the present invention relates to an electrode for active oxygen species and a sensor for measuring the concentration of active oxygen species which can be applied to *in vivo* measurement without using a large amount of enzymes and without causing a problem of enzyme deactivation.

### BACKGROUND ART

Superoxide anion radicals (O₂⁻·), which are active oxygen species, are produced in vivo by oxidation of xanthine, hypoxanthine, and the like into uric acid by xanthine-xanthine oxidase (XOD), reduction of oxygen by hemoglobin, and the like. The superoxide anion radicals have an important role in the synthesis of physiologically active substances, bactericidal action, senility, and the like. Various active oxygen species derived from the superoxide anion radicals are reported to cause various diseases such as cancer. Therefore, measuring the concentration of the active oxygen species including superoxide anion radicals in the living body is believed to be important for specifying these various diseases.

When there is no substrate, these superoxide anion radicals become hydrogen peroxide (H₂O₂) and oxygen molecules (O₂) by a dismutation reaction as shown in the formula (1). This dismutation reaction consists of formation of HO₂· by the addition of a proton to the superoxide anion radicals, formation of hydrogen peroxide and oxygen molecules by the reaction of HO₂· with oxygen molecules, and formation of hydrogen peroxide and oxygen molecules by collision of HO₂· radicals (formulas (1)-(4)).

2H⁺ + 2O₂⁻ · → H₂O₂ + O₂ (1)

H⁺ + O₂⁻· → HO₂ (2)

HO₂· + O₂⁻· + H⁺ → H₂O₂ + O₂ (3)

HO₂· + HO₂· → H₂O₂ + O₂ (4)

In this reaction system, the superoxide anion radical functions as an electron acceptor (oxidizing agent), an electron donator (reducing agent), and a hydrogen ion acceptor (base). The former two functions have been applied to measuring the concentration of superoxide anion radicals. For example, the concentration of superoxide anion radicals was measured using the reaction for converting ferricytochrome c (trivalent) into ferrocytochrome c (divalent), the reaction for producing blue formazan from nitroblue tetrazolium (NBT), and the reaction for reducing tetranitromethane (TNM). All of these reactions were carried out on an *in vitro* basis. Chen et.al. describes an electrochemical superoxide sensor based on a hemin modified graphite electrode.

On the other hand, a method for quantitatively measuring the concentration of superoxide anion radicals *in vivo* has been investigated. For example, McNeil et al., Tariov et al., and Cooper et al. reported that the concentration of superoxide anion radicals can be electrochemically determined by preparing an enzyme electrode (a cytochrome c-immobilized electrode) by modifying the surface of a gold or platinum electrode with an enzyme, N-acetylcysteine, and immobilizing a protein such as cytochrome c, which is a metal protein having an iron complex referred to as hem as an oxidation-reduction center, via an S-Au bond (C. J. McNeil et al., Free Radical Res. Commun., 7, 89 (1989) ; M. J. Tariov et al., J. Am. Chem. Soc. 113, 1847 (1991) ; and J. M. Cooper, K. R. Greenough and C. J. McNeil, J. Electroanal. Chem., 347, 267 (1993)).

The method is based on the following measurement principle. That is, cytochrome c (trivalent) (cyt.c(Fe³⁺)) reacts with superoxide anion radicals and is reduced to cytochrome c (divalent) (cyt.c (Fe²⁺)) according to the reaction formula (5) . Next, cytochrome c (divalent) reduced with O₂⁻ is electrochemically reoxidized according to the reaction formula (6). The oxidation current generated in this reaction is measured, whereby the concentration of the superoxide anion radicals are quantitatively determined in an indirect manner.

cyt.c(Fe³⁺) + O₂⁻ → cyt.c(Fe²⁺) + O₂ (5)

cyt.c(Fe²⁺) → cyt.c(Fe³⁺) + e- (6)

However, since cytochrome c is an electron transfer protein which is present in vivo on intracellular mitochondrial membranes, a large number of cells (e.g. 10⁵-10⁶ cells) is required to form an electrode on which cytochrome c is immobilized in an amount sufficient for the measurement. In addition, the enzyme used is deactivated in several days. Therefore, development of an electrode that can detect active oxygen species such as superoxide anion radicals without requiring a large amount of enzymes and without causing the problem of enzyme deactivation has been desired.

### DISCLOSURE OF THE INVENTION

In view of this situation, the inventors of the present invention have conducted extensive studies to obtain an electrode which can detect active oxygen species such as superoxide anion radicals by an oxidation-reduction reaction. As a result, the inventors have found that an electrode produced by forming a polymer membrane of a metal porphyrin complex, formed by introducing a metal atom into the center of a porphyrin compound, on the surface of a conductive component does not require a large amount of enzymes, is free from the problem of deactivation, and can be applied to detecting active oxygen species and measuring their concentration.

Specifically, the present invention provides an electrode for active oxygen species comprising a conductive component with a polymer membrane of a metal porphyrin complex formed on the surface.

The present invention further provides a sensor for measuring the concentration of active oxygen species comprising an electrode for active oxygen species comprising a conductive component with a polymer membrane of a metal porphyrin complex formed on the surface, a counter electrode, and a reference electrode.

Furthermore, the present invention provides a method for detecting active oxygen species in a sample comprising measuring the current produced between the metal in the metal porphyrin polymer membrane and the active oxygen species using the above-described sensor.

### BRIEF DESCRIPTION OF THE DRAWINGS

Figure 1 is a drawing showing an example of the three-electrode cell used for preparing the electrode of the present invention.
Figure 2 is a drawing showing an example of the two-chamber three-electrode cell used for preparing the electrode of the present invention.
Figure 3 is a drawing showing an example of the needle-type electrode and the two-chamber three-electrode cell used for preparing the electrode of the present invention, wherein (A) is the two-chamber three-electrode cell, (B) is the entire needle-type electrode, and (C) is the tip of the needle-type electrode.
Figure 4 is a drawing showing an improved needle-type electrode used for preparing the electrode of the present invention. This electrode is an improvement of the needle-type electrode of Figure 3, wherein (A) shows the entire improved needle-type electrode and (B) is the tip of the improved needle-type electrode.
Figure 5 is a drawing showing an example of the measuring device used for measuring active oxygen species.
Figure 6 is a drawing showing an example of the measuring device used for measuring active oxygen species.
Figure 7 shows graphs of the UV-visible spectrum of H₂T3ThP (7(a)) and UV-visible spectrum of FeT3ThP (7(b)).
Figure 8 shows graphs of the UV-visible spectrum of H₂T2AmP (8 (a)) and UV-visible spectrum of FeT2AmP (8(b)).
Figure 9 is a graph showing a CV curve during electrolytic polymerization of FeT3ThP.
Figure 10 is a graph showing a CV curve during electrolytic polymerization in the preparation of the electrode of FeT2AmP.
Figure 11 is a graph showing the change over time in the oxidation current during addition of XOD in Comparative Product 1.
Figure 12 is a graph showing the change over time in the oxidation current during addition of XOD in Inventive Product 1.
Figure 13 is a graph showing the relation between the (degree of XOD activity)^{1/2} and the amount of current increase in Inventive Product 1 and Comparative Product 1.
Figure 14 is a drawing showing the change over time in the current when XOD in Inventive Product 4 was added to a concentration of 100 mU/ml.
Figure 15 is a drawing showing the relation between the amount of superoxide anion radicals and the amount of current change.

### BEST MODE FOR CARRYING OUT THE INVENTION

The electrode for active oxygen species of the present invention (hereinafter referred to as "electrode") comprises a conductive component with a polymer membrane of a metal porphyrin complex formed on the surface.

Any component commonly used for electrodes can be used as a conductive component for the electrode of the present invention without specific limitations. Examples include carbons such as glassy carbon (GC), graphite, pyrolytic graphite (PG), highly oriented pyrolytic graphite (HOPG), and activated carbon, noble metals such as platinum, gold, and silver, and In₂O₃/SnO₂ (ITO). Of these, glassy carbon is particularly preferable in view of economical efficiency, processability, lightweight, and the like. There are no specific limitations to the shape of the conductive component, inasmuch as such a shape is usable as an electrode. Various shapes such as a cylinder, square pillar, needle, and fiber can be used. A needle-like shape is preferable for measuring the concentration of active oxygen species in vivo, for example.

A polymer membrane of a metal porphyrin complex is formed on the surface of the conductive component in the present invention. As examples of the metal porphyrin complex used for producing the polymer membrane, the compounds of the following formula (I) or (II) can be given. 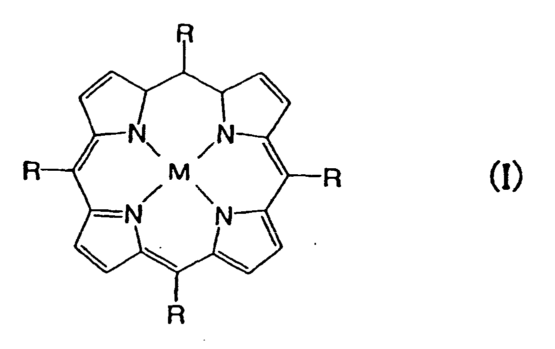 wherein M is a metal selected from the group consisting of iron, manganese, cobalt, chromium, and iridium, at least one of the four Rs is a group selected from the group consisting of a thiofuryl group, pyrrolyl group, furyl group, mercaptophenyl group, aminophenyl group, and hydroxyphenyl group, and the other Rs represent any one of these groups, an alkyl group, an aryl group, or hydrogen. 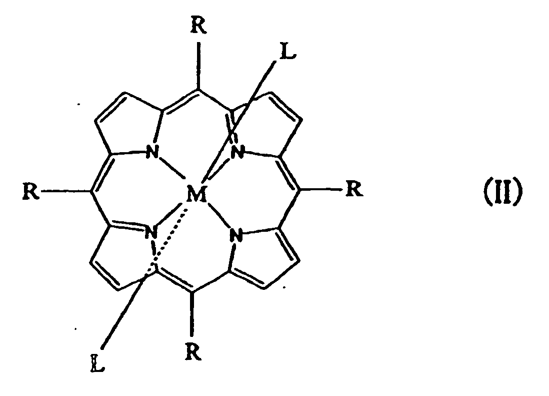 wherein M and R are the same as defined above, at least one of the two Ls is a nitrogen-containing axial ligand such as imidazole and its derivative, pyridine and its derivative, aniline and its derivative, histidine and its derivative, and trimethylamine and its derivative, a sulfur-containing axial ligand such as thiophenol and its derivative, cysteine and its derivative, and methionine and its derivative, or an oxygen-containing axial ligand such as benzoic acid and its derivative, acetic acid and its derivative, phenol and its derivative, aliphatic alcohol and its derivative, and water, and the other L is any one of these axial ligands or a group without a ligand.

The metal porphyrin complex represented by the above formula (I) or formula (II) is a complex compound in which a metal atom is coordinated to a porphyrin compound. This porphyrin compound is a cyclic compound formed from four pyrrole rings of which the four methine groups are bonded together at the α-position and the four nitrogen atoms are positioned face-to-face toward the center. A complex compound (a metal porphyrin complex) can be formed by inserting a metal atom into the center. To form this compound, a conventional method for producing a metal complex such as a method of introducing a metal atom into the center of porphyrin using metalation, for example, can be used. In the present invention, various metals such as iron, manganese, cobalt, chromium, and iridium can be used as the metal introduced into the center of the porphyrin compound.

A suitable metal atom can be selected according to the type of active oxygen species to be measured. For example, iron, manganese, cobalt, and the like are preferably used when superoxide anion radicals are measured; iron, cobalt, manganese, chromium, iridium, and the like are preferably used when molecular oxygen is measured; iron, manganese, and the like are preferably used when hydrogen peroxide is measured; and iron, manganese, and the like are preferably used when ·OH, NO, ONOO⁻, and the like are measured.

The porphyrin compound used in the present invention is preferably a porphyrin compound of which at least one of the 5, 10, 15, and 20 positions according to the position numbering of the IUPAC nomenclature is substituted with a thiofuryl group, pyrrolyl group, furyl group, mercaptophenyl group, aminophenyl group, hydroxyphenyl group, or the like, and the other positions are substituted with any one of these groups, an alkyl group, an aryl group, or hydrogen. The following compounds can be given as specific examples:
5,10,15,20-tetrakis(2-thiofuryl)porphyrin,
5,10,15,20-tetrakis(3-thiofuryl)porphyrin,
5,10,15,20-tetrakis(2-pyrrolyl)porphyrin,
5,10,15,20-tetrakis(3-pyrrolyl)porphyrin,
5,10,15,20-tetrakis(2-furyl)porphyrin,
5,10,15,20-tetrakis(3-furyl)porphyrin,
5,10,15,20-tetrakis(2-mercaptophenyl)porphyrin,
5,10,15,20-tetrakis(3-mercaptophenyl)porphyrin,
5,10,15,20-tetrakis(4-mercaptophenyl)porphyrin,
5,10,15,20-tetrakis(2-aminophenyl)porphyrin,
5,10,15,20-tetrakis(3-aminophenyl)porphyrin,
5,10,15,20-tetrakis(4-aminophenyl)porphyrin,
5,10,15,20-tetrakis(2-hydroxyphenyl)porphyrin,
5,10,15,20-tetrakis(3-hydroxyphenyl)porphyrin,
5,10,15,20-tetrakis(4-hydroxyphenyl)porphyrin,
[5,10,15-tris(2-thiofuryl)-20-mono(phenyl)]porphyrin,
[5,10,15-tris(3-thiofuryl)-20-mono(phenyl)]porphyrin,
[5,10-bis(2-thiofuryl)-15,20-di(phenyl)]porphyrin,
[5,10-bis(3-thiofuryl)-15,20-di(phenyl)]porphyrin,
[5,15-bis(2-thiofuryl)-10,20-di(phenyl)]porphyrin,
[5,15-bis(3-thiofuryl)-10,20-di(phenyl)]porphyrin,
[5-mono(2-thiofuryl)-10,15,20-tri(phenyl)]porphyrin, and
[5-mono(3-thiofuryl)-10,15,20-tri(phenyl)]porphyrin.

Among the ligands represented by L in the compound of the formula (II), as examples of the imidazole derivative, methylimidazole, ethylimidazole, propylimidazole, dimethylimidazole, and benzimidazole can be given; as examples of the pyridine derivative, methylpyridine, methyl pyridylacetate, nicotinamide, pyridazine, pyrimidine, pyrazine, and triazine can be given; as examples of the aniline derivative, aminophenol and diaminobenzene can be given; as examples of the histidine derivative, histidine methyl ester, histamine, and hippuryl-histidyl-leucine can be given; as examples of the trimethylamine derivative, triethylamine and tripropylamine can be given; as examples of the thiophenol derivative, thiocresol, mercaptophenol, mercaptobenzoic acid, aminothiophenol, benzene dithiol, and methylbenzene dithiol can be given; as examples of the cysteine derivative, cysteine methyl ester and cysteine ethyl ester can be given; as examples of the methionine derivative, methionine methyl ester and methionine ethyl ester can be given; as examples of the benzoic acid derivative, salicylic acid, phthalic acid, isophthalic acid, and terephthalic acid can be given; as examples of the acetic acid derivative, trifluoroacetic acid, mercaptoacetic acid, propionic acid, and butyric acid can be given; as examples of the phenol derivative, cresol and dihydroxybenzene can be given; and as examples of the aliphatic alcohol derivative, ethyl alcohol and propyl alcohol can be given.

Various polymerization methods such as electrolytic polymerization, solution polymerization, and heterogeneous polymerization can be used in the present invention to form a polymer membrane of a metal porphyrin complex on the surface of the conductive component. Of these, electrolytic polymerization is preferable. Specifically, the polymer membrane of a metal porphyrin complex can be formed on the surface of the conductive component by polymerization. Polymerization is carried out by two-electrode (working electrode and counter electrode) electrolysis or three-electrode (working electrode, counter electrode, and reference electrode) electrolysis, including three-electrode constant potential electrolysis, three-electrode constant current electrolysis, three-electrode reversible potential sweep electrolysis, and three-electrode pulse electrolysis, using a suitable supporting electrolyte such as tetrabutylammonium perchlorate (TBAP: Bu₄NClO₄), tetrapropylammonium perchlorate (TPAP: Pr₄NClO₄), or tetraethylammonium perchlorate (TEAP: Et₄NClO₄) in an organic solvent such as dichloromethane, chloroform, or carbon tetrachloride, using the conductive component as the working electrode, an insoluble electrode such as a noble-metal electrode (e.g. Pt electrode), a titanium electrode, a carbon electrode, or a stainless steel electrode as the counter electrode, and a saturated calomel electrode (SCE), a silver-silver chloride electrode, or the like as the reference electrode.

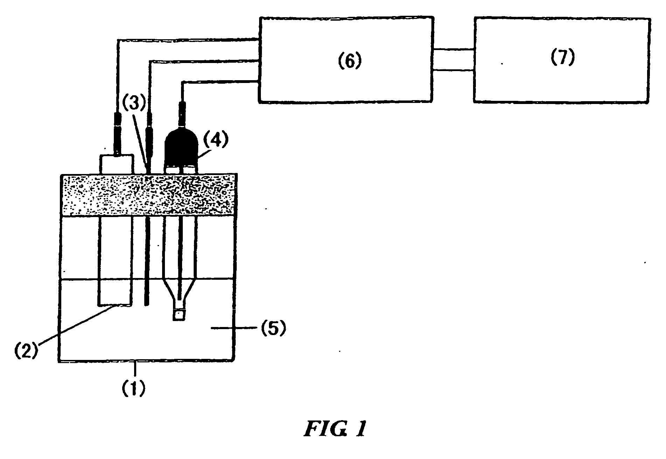

The electrolytic polymerization is preferably carried out by reversible potential sweep electrolysis or the like using a three-electrode cell as shown in Figure 1, for example. In Figure 1, 1 indicates a cell container; 2, a conductive component; 3, a counter electrode; 4, a reference electrode; 5, a metal porphyrin complex solution; 6, a potentiostat; and 7, an X-Y recorder.

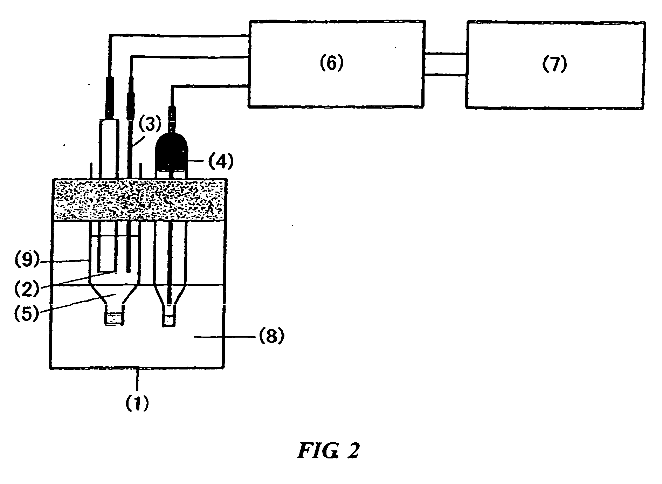

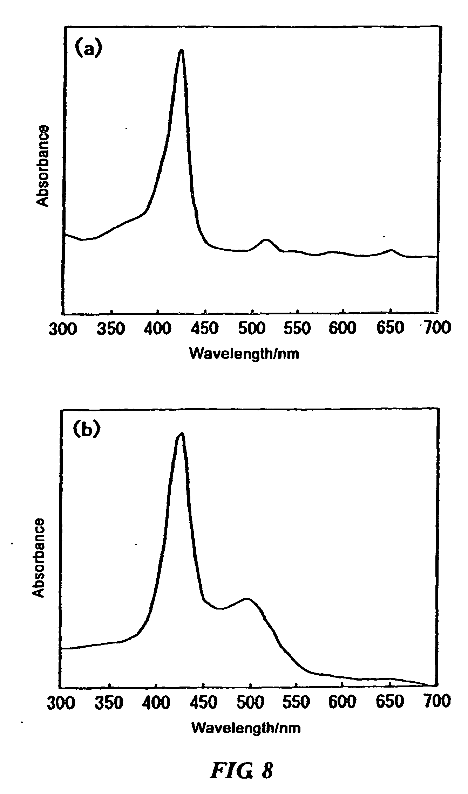

When using a high concentration metal porphyrin complex solution, a two-chamber three-electrode cell as shown in Figure 2, for example, may be used. In Figure 2, numerals 1-7 indicate the same items as in Figure 1, 8 indicates an electrolyte solution, and 9 is a sample vial.

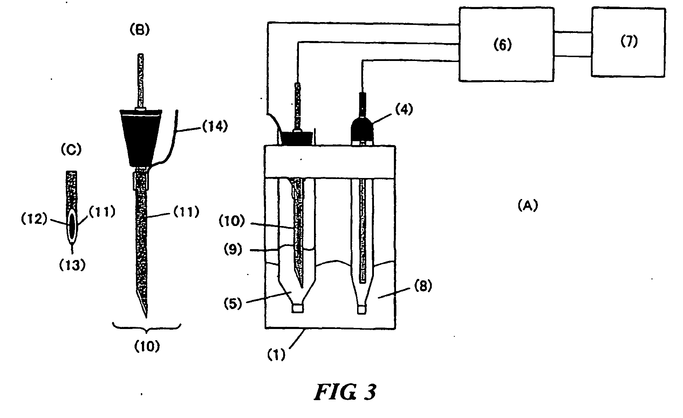

To produce a simplified electrode for active oxygen species (a needle-type electrode), a needle-type electrode 10 and a three-electrode cell as shown in Figures 3 (A) and 3 (B), for example, may be used. In Figure 3, 1 indicates a cell container; 4, a reference electrode; 5, a metal porphyrin complex solution; 6, a potentiostat; 7, an X-Y recorder; 8, an electrolyte solution; 9, a sample vial; 10, a needle-type electrode; 11, a counter electrode; 12, a tip of the conductive component (metal porphyrin polymer membrane area); 13, an electrical insulating material; and 14 a counter electrode wire.

As shown in Figure 3 (C), this electrode employs a counter electrode 11 prepared by filling a small tube of an electrical insulating material 13 with the conductive component and covering this small tube with the metal used as the counter electrode. The electrode can be used as a needle-type electrode by forming a metal porphyrin polymer membrane on the surface at the tip 12 of the conductive component.

The thickness of the polymer membrane of the metal porphyrin complex is appropriately determined according to the type of the electrode and metal porphyrin complex and the type of active oxygen to be measured. A thickness of 1 µm or less is preferable from the viewpoint of electrode activity, modification stability, and the like.

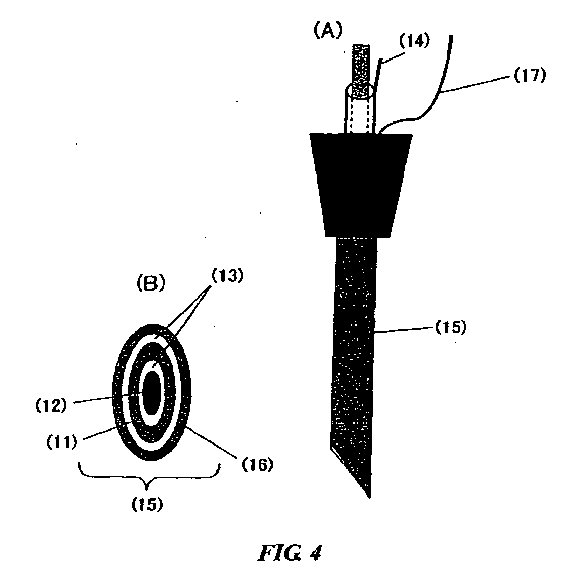

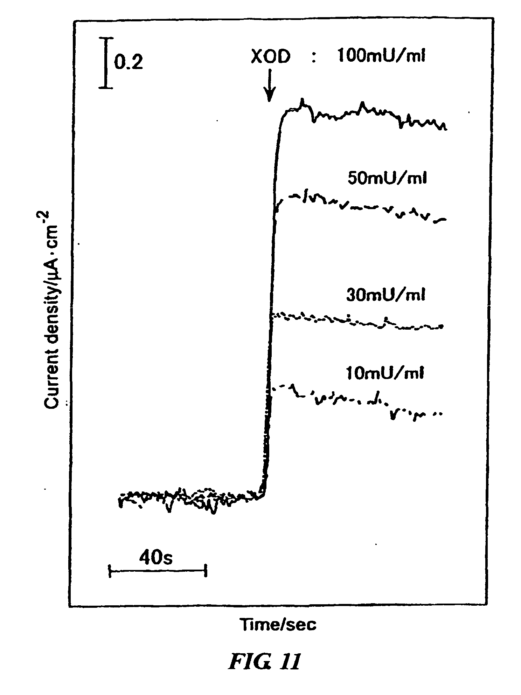

To produce a simplified electrode for active oxygen species (an improved needle-type electrode), based on the simplified electrode for active oxygen species (needle-type electrode) shown in Figure 3, with an objective of removing an unnecessary current in the living body, current noises, and the like and of improving sensitivity, signal/noise ratio (S/N ratio), and the like, an improved needle-type electrode 15 as shown in Figure 4(A) and a three-electrode cell as shown in Figure 3 (A) may be used, for example. In Figure 4, 11 indicates a counter electrode; 12, a tip of the conductive component (metal porphyrin polymer membrane area); 13, an electrical insulating material; 14, a counter electrode wire; 15, an improved needle-type electrode; 16, a ground; and 17, a ground wire.

As shown in Figure 4 (B), this electrode has a conductive component inserted in an electrical insulating material 13 (two-layer structure). The electrical insulating material 13 is placed in a counter electrode material 11 (three-layer structure), the counter electrode material 11 is housed in an electrical insulating material 13 (four-layer structure), and finally, the outside of the resulting small tube is coated with a material such as a metal capable of functioning as a ground (five-layer structure). The coating acts as the ground 16. The electrode can be used as an improved simplified electrode for active oxygen species (improved/needle-type electrode) by forming a metal porphyrin polymer membrane on the surface at the tip 12 of the conductive component.

The thickness of the polymer membrane of the metal porphyrin complex is appropriately determined according to the type of the electrode and metal porphyrin complex and the type of active oxygen to be measured. A thickness of 1 µm or less is preferable from the viewpoint of electrode activity, modification stability, and the like. This improved/needle-type electrode can also be used for measuring composite materials and the like. In such a case, it is possible to fabricate an electrode having a structure of up to ten or more layers. As the material for the ground, a noble metal such as platinum, gold, titanium, stainless steel, and silver, a corrosion-resistant alloy such as an iron-chromium alloy, carbon, or the like can be used. Since the ground is frequently used *in vivo,* a material with a high safety such as a noble metal (e.g. platinum, gold, silver), titanium, stainless steel, and carbon is preferable.

To use the electrode of the present invention for measuring active oxygen species, particularly for measuring the concentration of active oxygen species, it is preferable to combine the electrode with (1) a counter electrode and a reference electrode (three-electrode type) or (2) a counter electrode (two-electrode type). As the material for this counter electrode, a noble metal such as platinum, gold, and silver, titanium, stainless steel, a corrosion-resistant alloy such as an iron-chromium alloy, carbon, or the like can be used. Since the counter electrode is frequently used in vivo, a material with a high safety such as a noble metal (e.g. platinum, gold, silver), titanium, and carbon is preferable.

As the reference electrode, various reference electrodes such as a silver/silver chloride electrode and a mercury/mercuric chloride electrode can be usually used. A solid standard electrode can also be used.

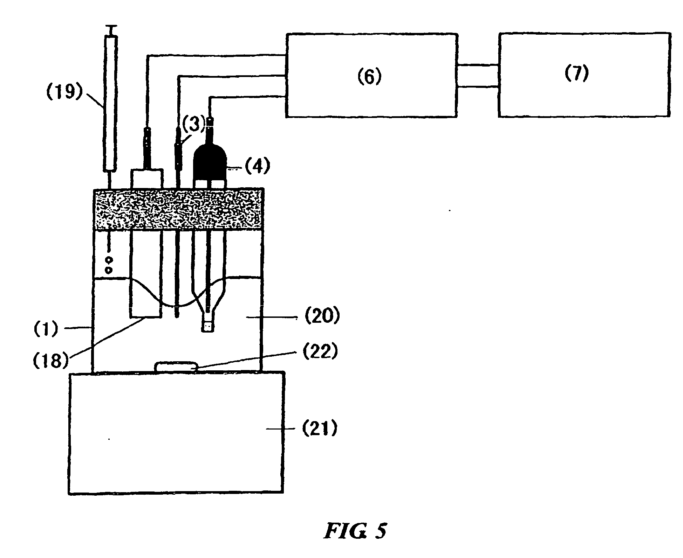

A specific example of the measuring device that can be used for measuring active oxygen species is shown in Figure 5. In Figure 5, numerals 1, 3, 4, 6, and 7 indicate the same items as in Figure 1, 18 indicates a measuring electrode (working electrode), 19 indicates a microsyringe, 20 indicates a solution to be measured, 21 indicates a magnetic stirrer, and 22 is a stirrer.

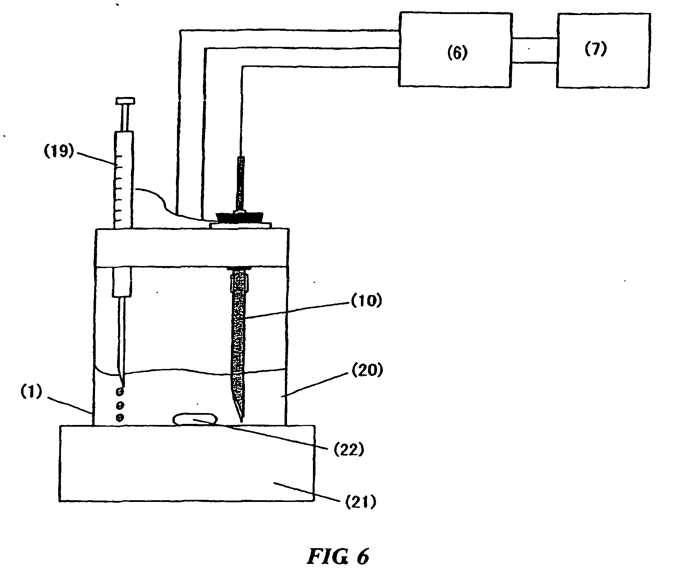

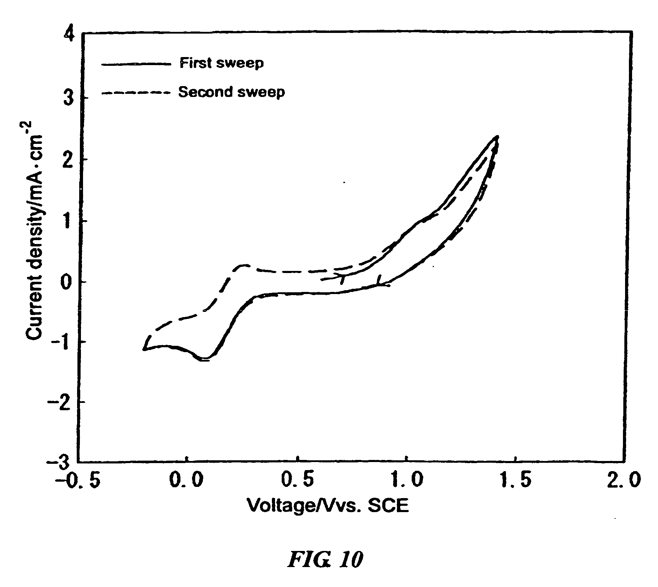

Another specific example of the measuring device used for measuring active oxygen species is shown in Figure 6. In Figure 6, numerals 1, 6, 7, and 19-22 indicate the same items as in Figure 4 and 10 indicates a needle-type electrode.

Although the electrode for active oxygen species of the present invention can be used as an electrode for detecting active oxygen species such as superoxide anion radicals using the above-described device, the electrode can also be used as a sensor for measuring the concentration of active oxygen species by using in combination with (1) a counter electrode and a reference electrode (three-electrode type) or (2) a counter electrode (two-electrode type). If the sensor for measuring the concentration of active oxygen species of this configuration is used in a system containing superoxide anion radicals, for example, the metal in the metal porphyrin complex forming the polymer membrane is reduced by the superoxide anion radicals. For example, if the metal is iron, Fe³⁺ is reduced to Fe²⁺ by the superoxide anion radicals (formula (7)).

If the Fe²⁺ reduced by the superoxide anion radicals is electrochemically reoxidized (formula (8)) while maintaining the electrode for measuring the concentration at a potential (in the case of the three-electrode type (1)) or a voltage (in the case of the two-electrode type (2)) to a degree at which Fe²⁺ can be oxidized, the current (oxidation current) flowing in this instance corresponds to the concentration of the superoxide anion radicals. Therefore, the concentration of the superoxide anion radicals dissolved in the sample solution can be quantitatively detected from the oxidation current. Specifically, the concentration of the superoxide anion radicals can be determined based on the same principle of the above formulas (5) and (6). The quantitative detection based on this principle is also possible for active oxygen species such as hydrogen peroxide and -OH, other active radical species such as NO and ONOO-, and the like.

Por (Fe³⁺) + O₂⁻· → Por (Fe²⁺) + O₂ (7)

Por (Fe²⁺) → Por (Fe³⁺) + e- (8)

wherein "Por" indicates porphyrin.

Since the electrode for active oxygen species of the present invention has a polymer membrane of a metal porphyrin complex on the surface of a conductive component, the electrode is remarkably strong and free from the problem of deactivation as compared with a conventional cytochrome c-immobilised electrode. In addition, since the polymer membrane of metal porphyrin is formed by electrolytic polymerization or the like, preparation of the electrode of the present invention is very easy as compared with a conventional electrode. The electrode of the present invention can be produced in a shape particularly suitable for application *in vivo,* for example, a needle-like shape.

In this manner, the electrode for active oxygen species of the present invention can not only detect active oxygen species such as superoxide anion radicals, hydrogen peroxide, and -OH and other active radical species (NO, ONOO-, etc.), but also quantitatively measure these active oxygen species by combining with a counter electrode and reference electrode in any environment including in vivo environment as well as *in vitro* environment. The electrode of the present invention therefore can be used widely in various fields.

Specifically, since various diseases can be specified by active oxygen species and other active radical species in vivo, a disease such as cancer can be detected by, for example, measuring the concentration of active oxygen species in blood.

On the other hand, with regard to the application in *in vitro* environment, decomposition conditions of food can be observed by measuring active oxygen species and their concentration in food. Water pollution conditions can also be observed by measuring active oxygen species and their concentration in tap water and sewage water.

Furthermore, the concentrations of superoxide anion radicals and superoxide dismutase (SOD), which is an enzyme with a function of eliminating the anions, can be measured by determining the extinction degree of the superoxide anion radicals when a sample containing the SOD is added.

### EXAMPLES

The present invention will be described in more detail by reference examples, examples, and test examples, which should not be construed as limiting the present invention.

### Reference Example 1

### Synthesis of 5,10,15,20-tetrakis(3-thiophenyl)porphyrin (H₂T3ThP)

A 100 ml round bottom flask was charged with 50 ml of proprionic acid, 2.0 ml of 3-thiophenecarbaldehyde, and 1.4 ml of pyrrole. The mixture was refluxed for one hour at 160°C while stirring. After refluxing, the reaction product was allowed to cool to room temperature, further cooled with ice, and added to 200 ml of cold methanol. The mixture was filtered by suction. The filtrate was washed with methanol and purified using silica gel chromatography (developing solvent: chloroform). The solvent was evaporated to dryness and the solid was recrystallized and dried under reduced pressure to obtain H₂T3ThP as black powder crystals (yield: 0.63 g, 19%). The product was identified using a UV-visible spectrum photometer (UV-2100, manufactured by Shimadzu Corp.) and by ¹H-NMR measurement. The results are shown in Tables 1 and 2.

### Reference Example 2

### Synthesis of 5,10,15,20-tetrakis(2-aminophenyl)porphyrin (H₂T2AmP)

A 2 L four-necked flask equipped with a reflux condenser was charged with 500 ml of propionic acid. After addition of 25 g of 2-nitrobenzaldehyde, the mixture was heated while refluxing at 110°C with stirring. 12 ml of pyrrole was added and the mixture was refluxed at the boiling point for 30 minutes. After addition of 50 ml of chloroform, the mixture was cooled with ice and filtered by suction. The filtrate was washed with 400 ml of chloroform and dried under reduced pressure (100°C, six hours, 0.1 kPa) to obtain a precursor 5,10,15,20-tetrakis (2-nitrophenyl) porphyrin (H₂T2NO₂P) as black purple crystals (yield: 5.0 g, 14%).

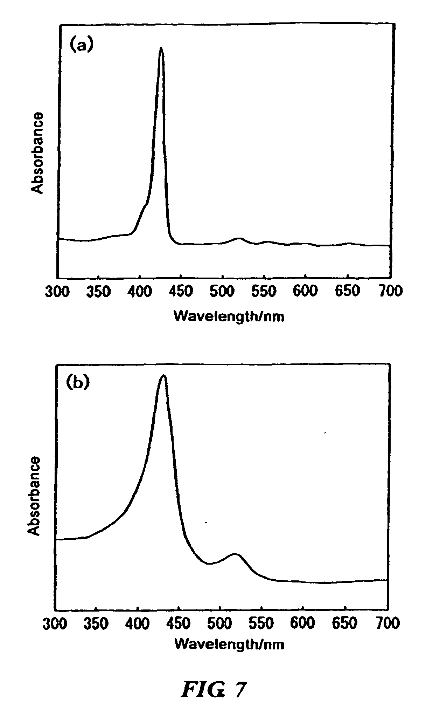

A 2 L four-necked flask was charged with 300 ml of 12 N HCl. After addition of 5.0 g of 5,10,15,20-tetrakis (2-nitrophenyl) porphyrin (H₂T2NO₂P) synthesized as described above and 20.0 g of tin (II) chloride dihydrate, the mixture was heated at 65-70°C for 30 minutes. Aqueous ammonia was gradually added and the mixture was filtered by suction. The filtrate was dried under reduced pressure. A 2 L beaker was charged with the filtrate. The filtrate was extracted with 10 L of acetone and the extract was evaporated to dryness using an evaporator. The dry solid was dissolved in 2 L of chloroform. The solution was washed with aqueous ammonia and ion exchange water and dehydrated with anhydrous sodium sulfate. The solvent was evaporated to dryness using an evaporator and the solid was recrystallized and dried under reduced pressure to obtain H₂T2AmP as purple crystals (yield: 4.0 g, 90%). The product was identified using a UV-visible spectrum photometer (UV-2100, manufactured by Shimadzu Corp.) and by ¹H-NMR measurement in the same manner as in Reference Example 1. The results are shown in Tables 1 and 2. Figure 7(a) shows the W-visible spectrum of H₂T3ThP and Figure 8 (a) shows the W-visible spectrum of H₂T2AmP.

### (Identification results)

**TABLE 1**

| Porphyrin | δ_{H}(CDCl₃3/TMS, ppm) |
|---|---|
| 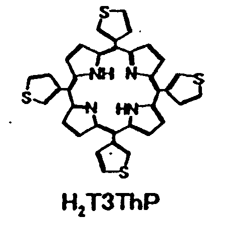 | -2.7(s,2H,pyrrole-NH) |
| | 7-7(t,4H,thiophene-H) |
| | 8.0(m.8H,thiophene-H) |
| | |
| | 8.9(s,8H,pyrrole-β-H) |
| 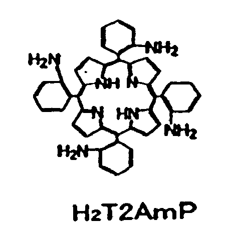 | -2.7(s,2H,pyrrole-NH) |
| | 4.0(s,8H,amino-H) |
| | 7.0-7.7(m,16H,phenyl-H) |
| | 8.9(s,8H,pyrrole-β-H) |

### (Identification results)

**TABLE 2**

| | λₘₐₓ(nm) | | | | |
|---|---|---|---|---|---|
| Porphyrin | Soret band | Q band | | | |
| H₂T3ThP | 422 | 519 | 556 | 594 | 651 |
| FeT3ThP | 425 | 516 | | | |
| H₂T2AmP | 420 | 516 | 549 | 590 | 652 |
| FeT2AmP | 425 | 493 | | | |

The results of identification for H₂T3ThP and H₂T2AmP in Table 1 confirmed peaks of protons forming the porphyrin ring and peaks specific to the porphyrin compounds. The peak of H₂T2AmP was confirmed at 4.0 ppm, which was assumed to be the peak of a proton forming an amino group. The results in Table 2 and Figures 7(a) and 8(a) confirmed the UV-visible spectra based on H₂T3ThP and H₂T2AmP. The above results confirmed that H₂T3ThP and H₂T2AmP were synthesized in Reference Examples 1 and 2.

### Reference Example 3

### Synthesis of metal porphyrin complex (1) (introduction of central metal (Fe) into H₂T3ThP by metalation)

A 50 ml three-necked flask was charged with 10 ml of 48% hydrobromic acid. After injection of nitrogen gas for 30 minutes, 100 mg of reduced iron was added. The mixture was stirred at 100°C until the reduced iron was dissolved. After the stirring, the solvent was evaporated under reduced pressure to obtain iron bromide anhydride as white powder.

250 mg of porphyrin (H₂T3ThP) prepared in Reference Example 1 and 200 ml of dimethylformamide (DMF) to which nitrogen gas was previously injected for 30 minutes were added to the product. The mixture was reacted in a nitrogen atmosphere for four hours. After the reaction, 200 ml of chloroform was added to the reaction product. The mixture was washed with ion exchange water, dehydrated with anhydrous sodium sulfate, and filtered. The solvent was evaporated using an evaporator and the solid obtained was purified using alumina column chromatography (developing solvent: chloroform/methanol = 20/1). After adding 48% hydrobromic acid to the eluate, the mixture was dehydrated with anhydrous sodium sulfate and filtered. The solvent was evaporated and the solid was recrystallized and dried under reduced pressure to obtain metal porphyrin (H₂T3ThP containing Fe at the center; hereinafter referred to as "FeT3ThP") as black crystals (yield: 230 mg, 84%).

### Reference Example 4

### Synthesis of metal porphyrin complex (2) (introduction of central metal (Fe) into H₂T2AmP by metalation)

Black crystals of a metal porphyrin complex containing Fe at the center of H₂T2AmP (hereinafter referred to as "FeT2AmP") were obtained in the same manner as in Reference Example 3, except for using 250 mg of H₂T2AmP (obtained in Reference Example 2) as a porpherin compound (yield: 224 mg, 83%). The products of Reference Examples 3 and 4 were identified using a UV-visible spectrum photometer. The results are shown in Figures 7 and 8 and Table 2.

Figure 7 (a) shows the UV-visible spectrum of H₂T3ThP and Figure 7(b) shows the UV-visible spectrum of FeT3ThP. Figure 8 (a) shows the UV-visible spectrum of H₂T2AmP and Figure 8 (b) shows the UV-visible spectrum of FeT2AmP. Table 3 shows the results of measuring the UV-visible spectra.

The porphyrin compounds in which a metal is not coordinated at the center (H₂T3ThP and H₂T2AmP) have a peak based on the conjugate ring at near 400 nm. The molecular extinction coefficient in the peak is 3.6-6.0 × 10⁵ M⁻¹ cm⁻¹. The peak is called a Soret band.

In addition, the porphyrin compounds have four peaks called Q bands of which the molecular extinction coefficient is 10⁴ M⁻¹ cm⁻¹ in the visible area. Introduction of a metal is confirmed generally by using spectral changes of the Q bands. Comparison of (a) with (b) in Figure 7 and Table 2 indicates that there are four peaks of Q bands in (a), whereas the number of peaks is reduced to one in (b). This is in agreement with a typical behavior when porphyrin forms a metal complex. Accordingly, it was confirmed that porphyrin formed a complex with iron, whereby a metal porphyrin complex was synthesized.

### Reference Example 5

### Synthesis of metal porphyrin complex (3) (introduction of central metal (Mn) into H₂T3ThP by metalation)

A 300 ml four-necked flask equipped with a reflux condenser with an argon balloon attached to the upper part, which can be heated over an oil bath and stirred using a magnetic stirrer, was charged with 100 ml of DMF containing 0.5 g of manganese acetate and 0.5 g of H₂T3ThP obtained in Reference Example 1. After saturation with argon, the mixture was refluxed at 140°C for one hour.

The resulting solution was poured into a separating funnel and washed with chloroform and ion exchange water. After addition of anhydrous magnesium sulfate, the mixture was dehydrated for one hour and filtered. The filtrate was evaporated to dryness using an evaporator.

The dry solid was separated and purified using column chromatography (filler: basic alumina, eluate: chloroform/methanol = 20/l). After the filtration, the filtrate was evaporated to dryness. The solid was dried under reduced pressure (0.1 kPa, 100°C) to obtain MnT3ThP as black purple crystals (yield: 0.36 g).

The UV-visible absorption spectrum was measured to confirm introduction of a metal. The absorption peaks of MnT3ThP were confirmed at 380 nm, 405 nm, 480 nm, 533 nm, 583 nm, and 623 nm, which differed from the above-described case of H₂T3ThP. Introduction of a metal was thus confirmed.

### Reference Example 6

### Synthesis of metal porphyrin complex having axial ligand (1) (synthesis of FeT3ThP to which 1-methylimidazole is coordinated)

FeT3ThP obtained in Reference Example 3 (0.018 g) and 1-methylimidazole (2-100 µl) (FeT3ThP : 1-methylimidazole = 1-50 (molar ratio)) were added to 0.5 ml of dichloromethane. The mixture was stirred while irradiating ultrasonic wave (15W) for five minutes or not irradiating ultrasonic wave for six hours or more.

The UV-visible absorption spectrum was measured to confirm coordination of a ligand. An absorption peak of the complex obtained by coordinating 1-methylimidazole to FeT3ThP was generated at 421 nm, which differed from the above-described case of FeT3ThP. Coordination of a ligand was thus confirmed.

The complex was evaporated to dryness using an evaporator and stored, or used for preparing an electrode for active oxygen species.

### Example 1

### Preparation of electrode for active oxygen species (1)

A glassy carbon (GC) electrode (diameter: 1.0 mm, manufactured by BAS Inc.) was polished using an alumina polishing agent (0.05 µm). After washing with water, the electrode was further washed with methanol. A polymer membrane was formed on the surface of this electrode by electrolytic polymerization using the following electrolytic solution and procedure to prepare a glassy carbon electrode with a polymer membrane of FeT3ThP formed on the surface (Inventive Product 1) and a glassy carbon electrode with a polymer membrane of FeT2AmP formed on the surface (Inventive Product 2).

### (Sample solution)

As a metal porphyrin complex, FeT3ThP or FeT2AmP synthesized in Reference Example 3 or 4 in a solution with a concentration of 0.05 M was used. As a solvent, (anhydrous) dichloromethane containing 0.1 M tetrabutylammonium perchlorate (Bu₄NClO₄/TBAP) as a supporting electrolyte was used. Oxygen dissolved in the solvent was removed using argon gas.

### (Procedure)

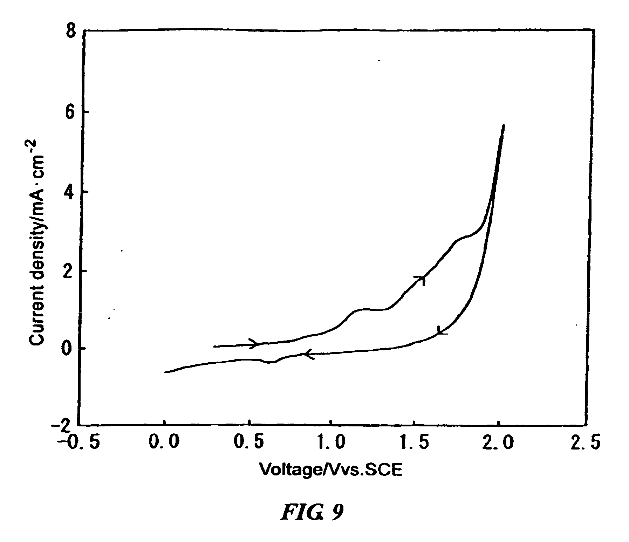

Electrolytic polymerization was carried out by reversible potential sweep electrolysis using a three-electrode cell having a configuration shown in Figure 1 (working electrode: GC, counter electrode: Pt line, reference electrode: SCE). The sweep range was 0 to 2.0 V for SCE in the case of preparing an electrode for active oxygen species using FeT3ThP; the range was -0.2 to 1.4 V for SCE in the case of preparing an electrode for active oxygen species using FeT2AmP. The sweep rate was 0.05 V/s in both cases. The number of times of sweep was once in the case of FeT3ThP and three times in the case of FeT2AmP. The cyclic voltammogram obtained in this electrolytic procedure (CV curve) was recorded in a X-Y recorder (manufactured by Riken Denshi Co., Ltd.). The results are shown in Figures 9 and 10.

Figure 9 shows a CV curve during electrolytic polymerization when preparing an electrode using FeT3ThP as a metal porphyrin complex. Based on this curve, it is assumed that cationic radicals of thiophene are produced at +1.74 V (for SCE). Since almost no cathode current due to reduction of the cationic radicals flows when reversing the potential sweep, it is assumed that the produced cationic radicals are immediately polymerized in the solution. The reduction peak at near 0.6 V (for SCE) is assumed to be a redox response of the polymer. The results confirmed that a polymer membrane of the metal porphyrin complex (FeT3ThP) was formed on the surface of the GC electrode.

Figure 10 shows a CV curve during electrolytic polymerization when preparing an electrode using FeT2AmP as a metal porphyrin complex. Based on this curve, it is assumed that cationic radicals of aniline (aminobenzene) are produced at +1.02 V (for SCE) . Since almost no cathode current due to reduction of the cationic radicals flows when reversing the potential sweep, it is assumed that the produced cationic radicals are immediately polymerized in the solution. The reduction peak at near 0.1 V (for SCE) is assumed to be a redox response of the polymer. The same peak was found in the second sweep. Further progress of the polymerization reaction was thus confirmed. The reduction peak at near 0.23 V (for SCE) is assumed to be a redox response of the polymer. The results confirmed that a polymer membrane of the metal porphyrin complex (FeT2AmP) was formed on the surface of the GC electrode.

### Example 2

### Preparation of electrode for active oxygen species (2)

A glassy carbon (GC) electrode surrounded by polyether ether ketone (PEEK), with only one end being exposed (PEEK diameter: 3 mm, GC diameter: 1 mm, end area: 0.0079 cm²; the other end made of brass) was provided. The GC end of the elecrtrode was polished sequentially by 6 µm polishing diamond and 1 µm polishing diamond. After finish polishing on an alumina polishing pad using an alumina polishing agent (0.05 µm), the electrode was washed with ion exchange water and acetone.

0.0037 g of FeT2AmP obtained in Reference Example 4 and 0.171 g of TBAP were put into a 5 ml measuring flask. Acetonitrile was added to the mixture to make the total volume 5 ml, thereby obtaining a sample solution.

The sample solution was put into a cell vial. A three-electrode electrochemical cell with a GC electrode as a working electrode shown in Figure 1 was fabricated. The atmosphere was replaced with argon.

The potential sweep was carried out three times in the potential sweep range of -0.3 to 1.0 V for Ag/Ag⁺ at a potential sweep rate of 200 mV/sec. The sweep initiation potential and the sweep termination potential were 0 V for Ag/Ag⁺. The sweep was carried out first in the negative direction. After the sweep, the cell was washed sequentially with acetonitrile and ion exchange water to prepare a glassy carbon electrode with a polymer membrane of FeT2AmP formed on the surface (Inventive Product 3).

### Example 3

### Preparation of electrode for active oxygen species (3)

0.171 g of TBAP was put into a 5 ml measuring flask. Dichloromethane was added to make the total volume 5 ml, thereby obtaining an electrolytic solution.

0.0182 g of FeT2AmP obtained in Reference Example 4, 0.0171 g of TBAP, and 0.5 ml of dichloromethane were added to a sample vial, while the tip of the sample vial sealed with vycor glass was immersed in the electrolytic solution.

A Pt counter electrode and the GC electrode used in Example 2 were put into the sample vial and an Ag/Ag⁺ electrode was disposed on the outer side of the sample vial as shown in Figure 2 to fabricate a two-chamber three-electrode electrochemical cell. The atmosphere in the two chambers was replaced with argon.

The potential sweep was carried out three times in the potential sweep range of -0.3 to 2.5 V for Ag/Ag⁺ at a potential sweep rate of 50 mV/sec. The sweep initiation potential was 0 V for Ag/Ag⁺ and the sweep termination potential was -0.3 V for Ag/Ag⁺. The sweep was carried out first in the negative direction. After the sweep, the cell was washed sequentially with dichloromethane and ion exchange water to prepare a glassy carbon electrode with a polymer membrane of FeT2AmP formed on the surface (Inventive Product 4).

### Example 4

### Preparation of electrode for active oxygen species (4)

An electrode rod of glassy carbon (diameter: 0.28-0.30 mm) was introduced into a glass capillary (internal diameter: 0.3 mm). These products were introduced into a needle (made of platinum, stainless steel, or the like corresponding to about 18G injection needle). These products were joined and secured using an epoxy adhesive, acrylic adhesive, manicure, or the like. Next, the glassy carbon and the outside needle were respectively joined with a platinum electrode, stainless steel electrode, copper electrode, or the like via a conductive adhesive such as a silver paste or carbon paste. The tip was polished using a grinder to prepare a needle-type electrode with a three-layer structure as shown in Figure 3 (glassy carbon: working electrode, inner glass capillary: insulation part between working electrode and counter electrode, outer needle: counter electrode).

An electrolytic solution (5 ml) prepared in the same manner as in Example 3 was put into a cell container. A dichloromethane solution of FeT3ThP obtained in Reference Example 6 to which 1-methylimidazole was coordinated containing 0.0171 g of TBAP was added to a sample vial, while the tip of the sample vial sealed with vycor glass was immersed in the electrolytic solution.

The needle-type electrodes (working electrode and counter electrode) prepared as described above were put into the sample vial and an Ag/Ag⁺ electrode was disposed on the outer side of the sample vial as shown in Figure 3 to fabricate a two-chamber three-electrode electrochemical cell.

The cell was electrolytically polymerized using a reversible potential sweep method (potential sweep range: -0.1 to +2.0 V for Ag/Ag⁺, potential sweep rate: 10 to 500 mV/sec) and a constant potential method (potential: +2.0 V for Ag/Ag⁺) for 5-120 minutes. After the electrolytic polymerization, the cell was washed sequentially with dichloromethane and ion exchange water to prepare a needle-type electrode with a three-layer structure including a glassy carbon electrode with a polymer membrane of FeT3ThP to which 1-methylimidazole was coordinated on the surface (glassy carbon surface-modified by polymer membrane: working electrode, inner glass capillary: insulation part between working electrode and counter electrode, outer needle: counter electrode; Inventive Product 5).

### Comparative Example 1

### Preparation of cytochrome c-immobilized gold electrode

As a comparative product for the electrodes of the inventive products, a cytochrome c-immobilized gold electrode as an electrode for determining the concentration of superoxide anion radicals was prepared in the following manner.

A gold electrode (diameter: 1.6 mm, manufactured by BAS Inc.) was polished using an alumina polishing agent (0.05 µm) and washed with water. The electrode was electrochemically treated in 1 M H₂SO₄ and washed with water. Next, the electrode was immersed in 10 mM 3-mercaptopropionic acid (hereinafter abbreviated to MPA, manufactured by Aldrich Co.) (solvent: 10 mM phosphoric acid buffer solution (pH 7.0)) for 24 hours to prepare a MPA-modified gold electrode. After washing the electrode with water, the potential sweep was carried out in 0.48 mM cytochrome c (type IV from Horse Heart, manufactured by Sigma Co.) (solvent: 10 mM phosphoric acid buffer solution (pH 7.0)) for 30 minutes (sweep range: -0.4 to 0.4V (for Ag/AgCl), sweep rate: 0.05 V/s).
1-ethyl-3-(3-dimethylaminopropyl)carbodiimide (EDC, manufactured by Pierce Chemical Co.) was added to make the final concentration 0.5 M and the electrode was immersed in the mixture for two hours. The electrode was finally washed with a phosphoric acid buffer solution to obtain a cytochrome c-immobilized gold electrode (Comparative Product 1). The above phosphoric acid buffer solution was a mixture solution of disodium hydrogen phosphate and sodium dihydrogen phosphate. Water distilled once was used for adjusting the sample solution.

### Test Example 1

### Measurement of amount of superoxide anion radicals (1)

The amount of superoxide anion radicals was measured using Inventive Product 1 and Comparative Product 1.

First, the electrode of Inventive Product 1 or Comparative Product 1 as a working electrode and a Pt electrode as a counter electrode were put into a cell container and a silver/silver chloride electrode (Ag/AgCl) was used as a reference electrode to form a three-electrode cell for the test. An apparatus shown in Figure 5 was prepared using the three-electrode cell for the test at the center. In this test, the potential sweep range was set at -0.2 to 0.25 V (for Ag/AgCl) for the electrode of Comparative Product 1 and at -0.5 to 0.5 V (for Ag/AgCl) for the electrode of Inventive Product 1. Measurements were carried out using several sweep rates.

A 2 mM aqueous potassium hydroxide solution containing 14.4 mM xanthine (manufactured by Sigma Co.) and a 10 mM Tris buffer solution containing 10 mM potassium chloride (pH 7.5) were prepared. 0.365 ml of the former and 14.635 ml of the latter were mixed to prepare a 0.35 mM xanthine solution, which was used as a test solution. Oxygen dissolved in the test solution was removed using high-purity argon gas.

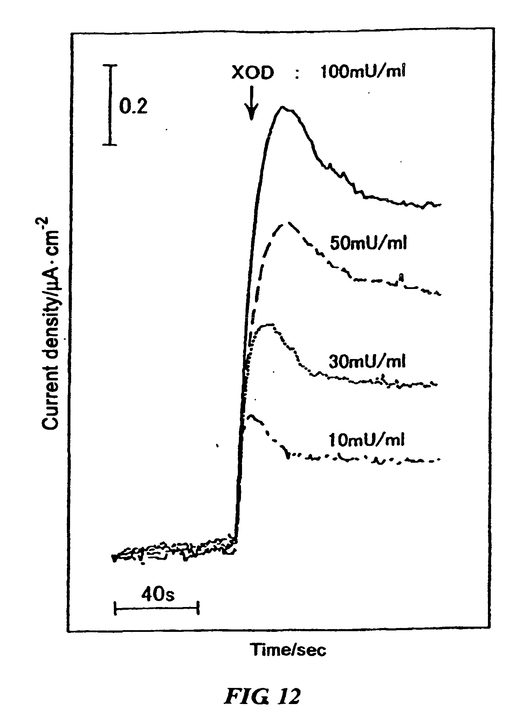

Second, the test solution was added to the three-electrode cell for the test. A potential of 0.2 V (for Ag/AgCl) which is sufficiently higher than the oxidation-reduction potential of each electrode was applied. Xanthine oxidase (XOD, Grade III from butter milk, manufactured by Sigma Co.) was added to the test solution to make the final concentration 0-100 mU/ml. The change over time in the oxidation current was recorded. The results are shown in Figure 12 for Inventive Product 1 and Figure 11 for Comparative Product 1. XOD had been dialyzed with a 10 ml phosphoric acid buffer solution (pH 7.0) before use. All measurements were carried out at room temperature.

XOD was added to xanthine to produce superoxide anion radicals dose-dependently. Figure 11 showing the change over time in the oxidation current during addition of XOD in Comparative Product 1 as the control indicates that the oxidation current rapidly increases immediately after the addition of XOD and is maintained almost at a constant value. Figure 12 showing the change over time in the oxidation current during addition of XOD in Inventive Product 1 indicates that the oxidation current rapidly increases immediately after the addition of XOD as in Figure 11 and the current once decreases and is maintained almost at a constant value and that the current value depends on the concentration of XOD, specifically, the amount of superoxide anion radicals.

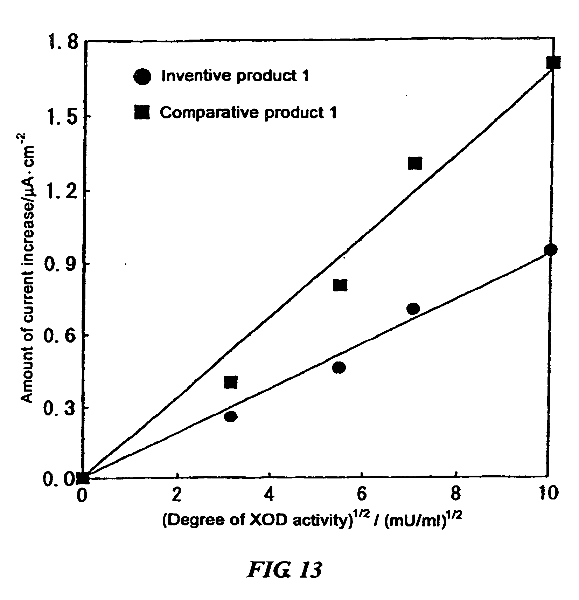

Figure 13 is a graph showing the relation between the (degree of XOD activity)^{1/2} and the amount of current increase when XOD is added in Inventive Product 1 and Comparative Product 1. The XOD activity was determined taking into consideration the values in the documents of Fridovich et al. and Cooper et al. (J. M. McCord and I. Fridovich, J. Boil. chem., 243, 5753 (1968), J. M. McCord and I. Fridovich, J. Boil. chem., 244, 6049 (1969), I. Fridorich, J. Boil. chem., 245, 4053 (1970), and J. M. Cooper, K. R. greenough, and C. J. McNeil, J. Electroanal. Chem., 347, 267 (1993)).

The results show that the amount of current increase in Inventive Product 1 is proportional to the (degree of XOD activity)^{½} and there is a linear relation between them in the same manner as in Comparative Product 1 used as the control. These facts confirmed that the concentration of superoxide anion radicals can be measured using the electrode for active oxygen species of the present invention.

### Test Example 2

### Measurement of amount of superoxide anion radicals (2)

The amount of superoxide anion radicals was measured using the electrode of Inventive Product 5.

The electrode of Inventive Product 5, specifically, a needle-type electrode with a three-layer structure (glassy carbon surface-modified with polymer membrane: working electrode, inner glass capillary: insulation part between working electrode and counter electrode, outer needle: counter electrode; Inventive Product 5) was measured using a two-electrode method. As a solution for measurement, a Tris buffer solution containing 0.15 mM xanthine (pH 7.5) was used. 0-100 mU/ml of XOD was added to the solution. The applied voltage was 0-1.0 V.

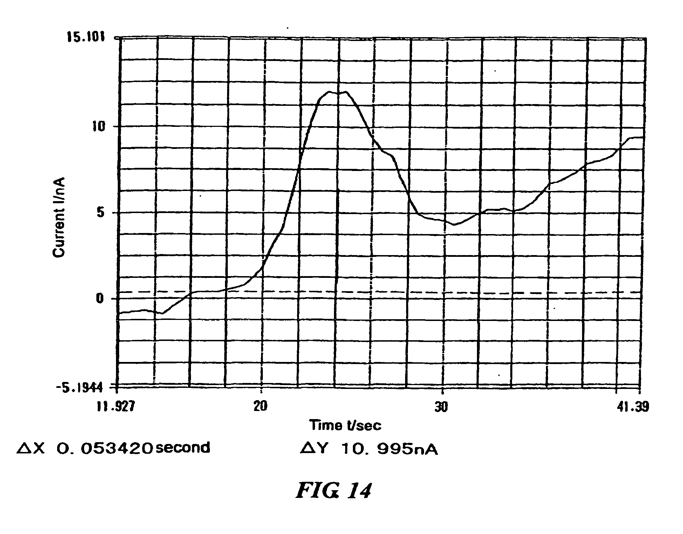

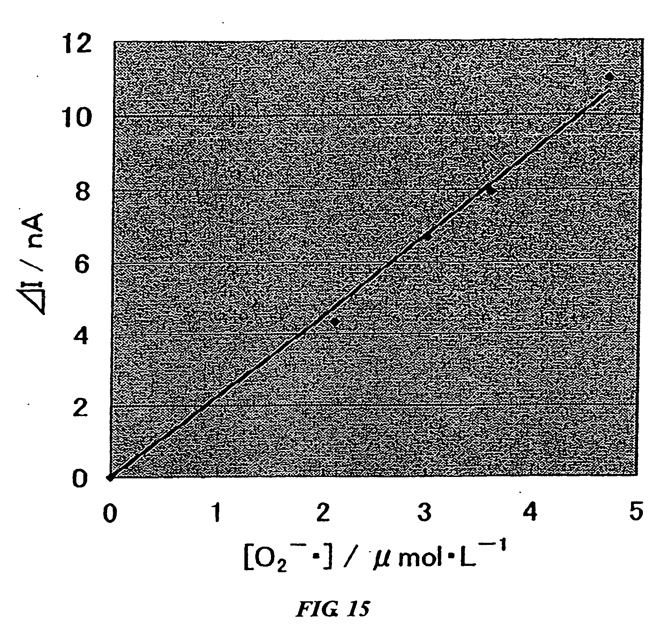

The change over time in the current when XOD was added to a concentration of 100 mU/ml at an applied voltage of +0.5 V is shown in Figure 14. A graph of the relation between the amount of superoxide anion radicals and the amount of current change prepared by the peak current value is shown in Figure 15.

The correlation coefficient between the amount of superoxide anion radicals and the amount of current change determined from Figure 15 was 0.995. This indicates that the electrode of the present invention can be effectively used for measuring the amount of superoxide anion radicals.

### INDUSTRIAL APPLICABILITY

The electrode for active oxygen species of the present invention can detect active oxygen species such as superoxide anion radicals, hydrogen peroxide, and ·OH and other active radical species (NO, ONOO-, etc.) in any environment including *in vivo* environment as well as *in vitro* environment. In addition, it is possible to quantitatively determine these active oxygen species and other active radical species by combining the electrode with a counter electrode or a reference electrode. The electrode thus can be widely used in various fields.

For example, if used in vivo, various diseases can be specified from active oxygen species and other active radical species in the living body.

On the other hand, if used *in vitro,* active oxygen species and their concentration in food can be measured, based on which decomposition conditions of the food can be judged. Water pollution conditions can also be observed by measuring active oxygen species and their concentration in tap water and sewage water.

## Claims

1. An electrode for active oxygen species comprising a conductive component with a polymer membrane of a metal porphyrin complex shown by the following formula (I) or (II) formed on the surface, 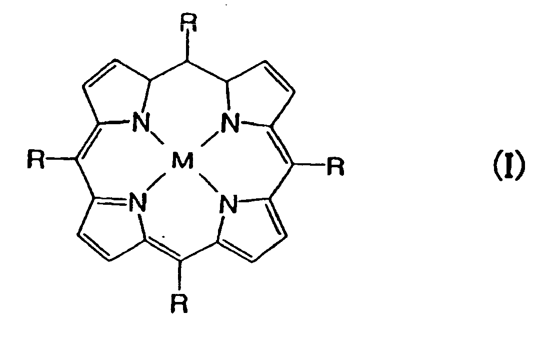 wherein M is a metal selected from the group consisting of iron, manganese, cobalt, chromium, and iridium, at least one of the four Rs is a group selected from the group consisting of a thiofuryl group, pyrrolyl group, furyl group, mercaptophenyl group, and aminophenyl group, and the other Rs represent any one of these groups, an alkyl group, an aryl group, or hydrogen, 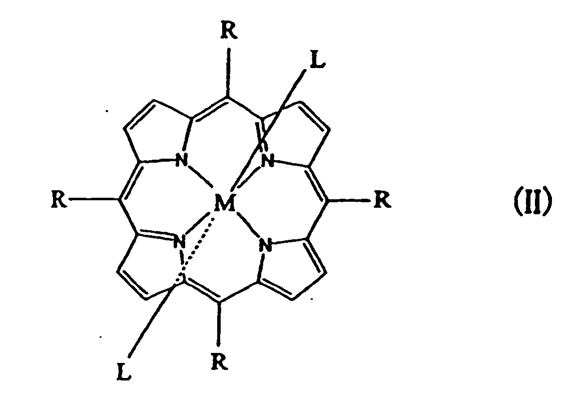 wherein M and R are the same as defined above, at least one of the two Ls is a nitrogen-containing axial ligand such as imidazole and its derivative, pyridine and its derivative, aniline and its derivative, histidine and its derivative, and trimethylamine and its derivative, a sulfur-containing axial ligand such as thiophenol and its derivative, cysteine and its derivative, and methionine and its derivative, or an oxygen-containing axial ligand such as benzoic acid and its derivative, acetic acid and its derivative, phenol and its derivative, aliphatic alcohol and its derivative, and water, and the other L is any one of these axial ligands or a group without a ligand.

2. The electrode according to claim 1, wherein the porphyrin compound forming the metal porphyrin complex is selected from the group consisting of
5,10,15,20-tetrakis(2-thiofuryl)porphyrin,
5,10,15,20-tetrakis(3-thiofuryl)porphyrin,
5,10,15,20-tetrakis(2-pyrrolyl)porphyrin,
5,10,15,20-tetrakis(3-pyrrolyl)porphyrin,
5,10,15,20-tetrakis(2-furyl)porphyrin,
5,10,15,20-tetrakis(3-furyl)porphyrin,
5,10,15,20-tetrakis(2-mercaptophenyl)porphyrin,
5,10,15,20-tetrakis(3-mercaptophenyl)porphyrin,
5,10,15,20-tetrakis(4-mercaptophenyl)porphyrin,
5,10,15,20-tetrakis(2-aminophenyl)porphyrin,
5,10,15,20-tetrakis(3-aminophenyl)porphyrin,
5,10,15,20-tetrakis(4-aminophenyl)porphyrin,
[5,10,15-tris(2-thiofuryl)-20-mono(phenyl)]porphyrin,
[5,10,15-tris(3-thiofuryl)-20-mono(phenyl)]porphyrin,
[5,10-bis(2-thiofuryl)-15,20-di(phenyl)]porphyrin,
[5,10-bis(3-thiofuryl)-15,20-di(phenyl)]porphyrin,
[5,15-bis(2-thiofuryl)-10,20-di(phenyl)]porphyrin,
[5,15-bis(3-thiofuryl)-10,20-di(phenyl)]porphyrin,
[5-mono(2-thiofuryl)-10,15,20-tri(phenyl)]porphyrin,and
[5-mono(3-thiofuryl)-10,15,20-tri(phenyl)]porphyrin.

3. The electrode according to claim 1 or claim 2, wherein the conductive component is inserted into a small tube prepared from an electrical insulating material, the outside of this small tube is covered with a material acting as a counter electrode such as a metal to form a counter electrode, a metal porphyrin polymer membrane is formed on the tip of the conductive component, and the electrode has a needle-like shape.

4. The electrode according to claim 1 or claim 2, wherein the conductive component is inserted into an electrical insulating material, the electrical insulating material is placed in a counter electrode material, the resulting counter electrode material is housed in an electrical insulating material, of which the outside is covered with a material acting as a ground, a metal porphyrin polymer membrane is formed on the tip of the conductive component, and the electrode has a needle-like shape.

5. The electrode according to any preceding claim, used for measuring superoxide anion radicals.

6. A sensor for measuring the concentration of active oxygen species comprising an electrode for active oxygen species comprising a conductive component with a polymer membrane of a metal porphyrin complex shown by the following formula (I) or (II) formed on the surface, a counter electrode, and a reference electrode, 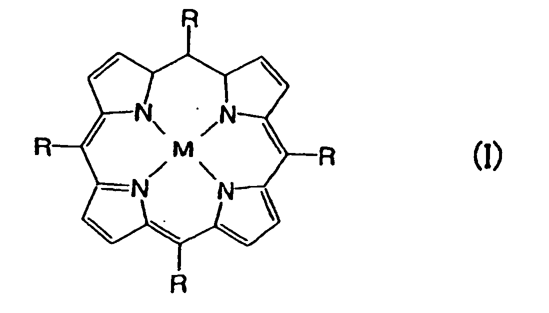 wherein M is a metal selected from the group consisting of iron, manganese, cobalt, chromium, and iridium, at least one of the four Rs is a group selected from the group consisting of a thiofuryl group, pyrrolyl group, furyl group, mercaptophenyl group, and aminophenyl group, and the other Rs represent any one of these groups, an alkyl group, an aryl group, or hydrogen, 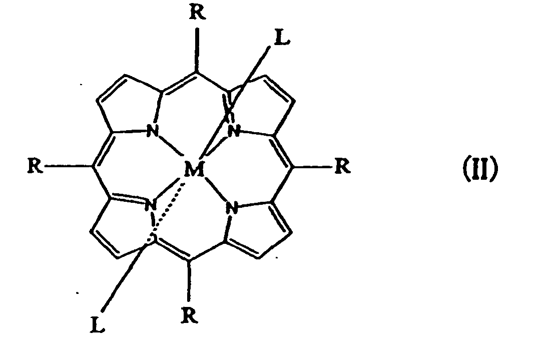 wherein M and R are the same as defined above, at least one of the two Ls is a nitrogen-containing axial ligand such as imidazole and its derivative, pyridine and its derivative, aniline and its derivative, histidine and its derivative, and trimethylamine and its derivative, a sulfur-containing axial ligand such as thiophenol and its derivative, cysteine and its derivative, and methionine and its derivative, or an oxygen-containing axial ligand such as benzoic acid and its derivative, acetic acid and its derivative, phenol and its derivative, aliphatic alcohol and its derivative, and water, and the other L is any one of these axial ligands or a group without a ligand.

7. The sensor according to claim 6, wherein the porphyrin compound forming the metal porphyrin complex is selected from the group consisting of
5,10,15,20-tetrakis(2-thiofuryl)porphyrin,
5,10,15,20-tetrakis(3-thiofuryl)porphyrin,
5,10,15,20-tetrakis(2-pyrrolyl)porphyrin,
5,10,15,20-tetrakis(3-pyrrolyl)porphyrin,
5,10,15,20-tetrakis(2-furyl)porphyrin,
5,10,15,20-tetrakis(3-furyl)porphyrin,
5,10,15,20-tetrakis(2-mercaptophenyl)porphyrin,
5,10,15,20-tetrakis(3-mercaptophenyl)porphyrin,
5,10,15,20-tetrakis(4-mercaptophenyl)porphyrin,
5,10,15,20-tetrakis(2-aminophenyl)porphyrin,
5,10,15,20-tetrakis(3-aminophenyl)porphyrin,
5,10,15,20-tetrakis(4-aminophenyl)porphyrin,
[5,10,15-tris(2-thiofuryl)-20-mono(phenyl)]porphyrin,
[5,10,15-tris(3-thiofuryl)-20-mono(phenyl)]porphyrin,
[5,10-bis(2-thiofuryl)-15,20-di(phenyl)]porphyrin,
[5,10-bis(3-thiofuryl)-15,20-di(phenyl)]porphyrin,
[5,15-bis(2-thiofuryl)-10,20-di(phenyl)]porphyrin,
[5,15-bis(3-thiofuryl)-10,20-di(phenyl)]porphyrin,
[5-mono(2-thiofuryl)-10,15,20-tri(phenyl)]porphyrin, and
[5-mono(3-thiofuryl)-10,15,20-tri(phenyl)]porphyrin.

8. The sensor according to claim 6 or claim 7, wherein an electrode is used, the electrode comprising a conductive component inserted into a small tube prepared from an electrical insulating material, the outside of this small tube being covered with a material acting as a counter electrode such as a metal to form a counter electrode, a metal porphyrin polymer membrane being formed on the tip of the conductive component, and the electrode having a needle-like shape.

9. The sensor according to claim 6 or claim 7, wherein an electrode is used, the electrode comprising a conductive component inserted into an electrical insulating material, the electrical insulating material being placed in a counter electrode material, the resulting counter electrode material being housed in an electrical insulating material, of which the outside is coated with a material acting as a ground, a metal porphyrin polymer membrane being formed on the tip of the conductive component, and the electrode having a needle-like shape.

10. The sensor according to any one of claims 6-9, used for measuring superoxide anion radicals.

11. A method for detecting active oxygen species in a sample comprising measuring a current produced by oxidation-reduction reaction between a metal in a metal porphyrin polymer membrane and active oxygen species using the sensor according to any one of claims 6-10.

12. The method according to claim 11, wherein the active oxygen species to be detected are superoxide anion radicals.

## Patentansprüche

1. Elektrode für aktive Sauerstoffspezies, umfassend eine leitende Komponente mit einer auf der Oberfläche gebildeten Polymermembran eines durch die folgende Formel (I) oder (II) gezeigten Metall-Porphyrin-Komplexes, 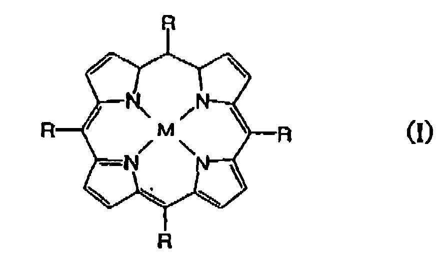 worin M ein Metall ist, ausgewählt aus der Gruppe, die aus Eisen, Mangan, Kobalt, Chrom und Iridium besteht, mindestens einer der vier Rs eine Gruppe ist, die ausgewählt ist aus der Gruppe, die aus einer Thiofurylgruppe, einer Pyrrolylgruppe, einer Furylgruppe, einer Mercaptophenylgruppe und einer Aminophenylgruppe besteht, und die anderen Rs irgendeine dieser Gruppen, eine Alkylgruppe, eine Arylgruppe oder Wasserstoff darstellen, 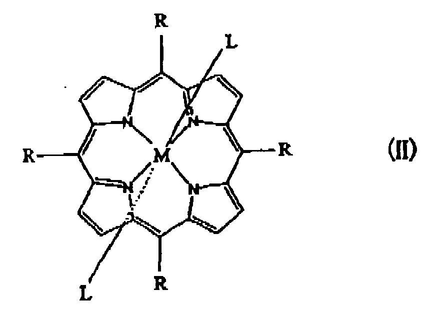 worin M und R gleich wie oben definiert sind, mindestens eines der beiden Ls ein Stickstoff-haltiger, axialer Ligand wie etwa Imidazol und dessen Derivat, Pyridin und dessen Derivat, Anilin und dessen Derivat, Histidin und dessen Derivat und Trimethylamin und dessen Derivat, ein Schwefel-haltiger, axialer Ligand wie etwa Thiophenol und dessen Derivat, Cystein und dessen Derivat und Methionin und dessen Derivat, oder ein Sauerstoff-haltiger, axialer Ligand wie Benzoesäure und dessen Derivat, Essigsäure und dessen Derivat, Phenol und dessen Derivat, aliphatischer Alkohol und dessen Derivat und Wasser ist, und das andere L irgendeines dieser axialen Liganden oder eine Gruppe ohne einen Liganden ist.

2. Elektrode gemäß Anspruch 1, wobei die den Metall-Porphyrin-Komplex bildende Porphyrinverbindung ausgewählt ist aus der Gruppe, die aus
5,10,15,20-tetrakis(2-Thiofuryl)porphyrin,
5,10,15,20-tetrakis(3-Thiofuryl)porphyrin,
5,10,15,20-tetxakis(2-Pyrrolyl)porphyrin,
5,10,15,20-tetrakis(3-Pyrrolyl)porphyrin,
5,10,15,20-tetrakis(2-Furyl)porphyrin,
5,10,15,20-tetrakis(3-Furyl)porphyrin,
5,10,15,20-tetrakis(2-Mercaptophenyl)porphyrin,
5,10,15,20-tetrakis(3-Mercaptophenyl)porphyrin,
5,10,15,20-tetrakis(4-Mercaptophenyl)porphyrin,
5,10,15,20-tetrakis(2-Aminophenyl)porphyrin,
5,10,15,20-tetrakis(3-Aminophenyl)porphyrin,
5,10,15,20-tetrakis(4-Aminophenyl)porphyrin,
[5,10,15-tris(2-Thiofuryl)-20-mono(phenyl)]porphyxin,
[5,10,15-tris(3-Thiofuryl)-20-mono(phenyl)]porphyrin,
[5,10-bis(2-Thiofuryl)-15,20-di(phenyl)lpoxphyrin,
[5,10-bis(3-Thiofuryl)-15,20-di(phenyl)]porphyrin,
[5,15-bis(2-Thiofuryl)-10,20-di(phenyl)jporphyxin,
[5,15-bie(3-Thiofuryl)-10,20-di(phenyl)]porphyxrin,
[5-mono(2-Thinfuryl)-10,15,20-tri(phenyl)]porphyrin und
[5-mono(3-Thiofuryl)-10,15,20-tri(phenyl)]porphyrin.

3. Elektrode gemäß Anspruch 1 oder 2, wobei die leitende Komponente in ein aus einem elektrisch isolierenden Material gefertigten kleinen Röhrchen eingeführt ist, die Außenseite dieses kleinen Röhrchens mit einem Material bedeckt ist, das als eine Gegenelektrode wirkt, wie einem Metall zum Bilden einer Gegenelektrode, eine Metall-Porphyrinpolymer-Membran auf der Spitze der leitenden Komponente gebildet ist, und die Elektrode eine nadel-förmige Gestalt aufweist.

4. Elektrode gemäß Anspruch 1 oder 2, wobei die leitende Komponente in ein elektrisch isolierendes Material eingeführt ist, das elektrisch isolierende Material in ein Material für eine Gegenelektrode eingebracht ist, das resultierende Material der Gegenelektrode in einem elektrisch isolierenden Material beherbergt wird, dessen Außenseite mit einem als Erdung wirkenden Material bedeckt ist, eine Metall-Porphyrinpolymer-Membran auf der Spitze der leitenden Komponente gebildet ist, und die Elektrode eine nadelförmige Gestalt aufweist.

5. Elektrode gemäß irgendeinem vorangehenden Anspruch, verwendet zum Messen von Superoxidanionradikalen.

6. Sensor zum Messen der Konzentration aktiver Sauerstoffspezies, der eine Elektrode für aktive Sauerstoffspezies mit einer leitenden Komponente mit einer auf der Oberfläche gebildeten Polymermembran eines durch die folgende Formel (I) oder (II) gezeigten Metall-Porphyrin-Komplexes, eine Gegenelektrode und eine Bezugselektrode aufweist, 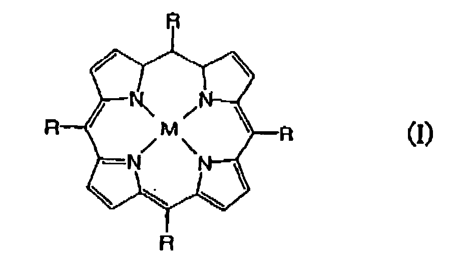 worin M ein Metall ist, ausgewählt aus der Gruppe, die aus Eisen, Mangan, Kobalt, Chrom und Iridium besteht, mindestens einer der vier Rs eine Gruppe ist, die ausgewählt ist aus der Gruppe, die aus einer Thiofurylgruppe, Pyrrolylgruppe, Furylgruppe, Mercaptophenylgruppe und Aminophenylgruppe besteht, und die anderen Rs irgendeine dieser Gruppen, eine Alkylgruppe, eine Arylgruppe oder Wasserstoff darstellen, 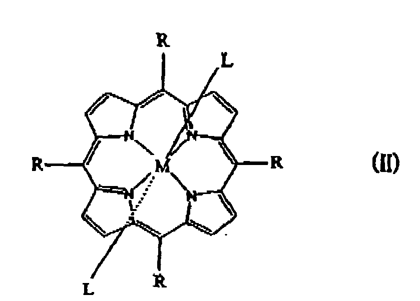 worin M und R gleich wie oben definiert sind, mindestens eines der beiden Ls ein Stickstoff-haltiger, axialer Ligand wie etwa Imidazol und dessen Derivat, Pyridin und dessen Derivat, Anilin und dessen Derivat, Histidin und dessen Derivat und Trimethylamin und dessen Derivat, ein Schwefel-haltiger, axialer Ligand wie etwa Thiophenol und dessen Derivat, Cystein und dessen Derivat und Methionin und dessen Derivat, oder ein Sauerstoff-haltiger, axialer Ligand wie Benzoesäure und dessen Derivat, Essigsäure und dessen Derivat, Phenol und dessen Derivat, aliphatischer Alkohol und dessen Derivat und Wasser ist, und das andere L irgendeines dieser axialen Liganden oder eine Gruppe ohne einen Liganden ist.

7. Sensor gemäß Anspruch 6, wobei die den Metall-Porphyrin-Komplex bildende Porphyrinverbindung ausgewählt ist aus der Gruppe, die aus
5,10,15,20-tetrakis(2-Thiofuryl)porphyrin,
5,10,15,20-tetrakis(3-Thiofuryl)porphyrin,
5,10,15,20-tetrakis(2-Pyrrolyl)porphyrin,
5,10,15,20-tetrakis(3-Pyrrolyl)porphyrin,
5,10,15,20-tetrakis(2-Furyl)porphyrin,
5,10,15,20-tetrakis(3-Furyl)porphyrin,
5,10,15,20-tetrakis(2-Mercaptophenyl)porphyrin,
5,10,15,20-tetrakis(3-Mercaptophenyl)porphyrin,
5,10,15,20-tetrakis(4-Mercaptophenyl)porphyrin,
5,10,25,20-tetrakis(2-Aminophenyl)porphyrin,
5,10,15,20-tetrakis(3-Aminophenyl)porphyrin,
5,10,15,20-tetrakis(4-Aminophenyl)porphyrin,
[5,10,15-tris(2-Thiofuryl)-20-mono(phenyl)]porphyrin,
[5,10,15-tris(3-Thiofuryl)-20-mono(phenyl)]porphyrin,
[5,10-bis(2-Thiofuryl)-15,20-di(phenyl)]porphyrin,
[5,10-bis(3-Thiofuryl)-15,20-di(phenyl)]porphyrin,
[5,15-bis(2-Thiofuryl)-10,20-di(phenyl)]porphyrin,
[5,15-bis(3-Thiofuryl)-10,20-di(phenyl)]porphyrin,
[5-mono(2-Thiofuryl)-10,15,20-tri(phenyl)]porphyrinund
[5-mono(3-Thiofuryl)-10,15,20-tri(phenyl)]porphyrin.

8. Sensor gemäß Anspruch 6 oder 7, wobei eine Elektrode verwendet wird, die Elektrode leitende Komponenten einschließt, die in ein aus einem elektrisch isolierenden Material gefertigten kleinen Röhrchen eingeführt ist, die Außenseite dieses kleinen Röhrchens mit einem Material bedeckt ist, das als eine Gegenelektrode wirkt, wie einem Metall zum Bilden einer Gegenelektrode, eine Metall-Porphyrinpolymer-Membran auf der Spitze der leitenden Komponente gebildet ist, und die Elektrode eine nadel-förmige Gestalt aufweist.

9. Sensor gemäß Anspruch 6 oder 7, wobei eine Elektrode verwendet wird, die Elektrode eine leitende Komponente einschließt, die in ein elektrisch isolierendes Material eingeführt ist, das elektrisch isolierende Material in ein Material für eine Gegenelektrode eingebracht ist, das resultierende Material der Gegenelektrode in einem elektrisch isolierenden Material beherbergt wird, dessen Außenseite mit einem als Erdung wirkenden Material bedeckt ist, eine Metall-Porphyrinpolymer-Membran auf der Spitze der leitenden Komponente gebildet ist, und die Elektrode eine nadelförmige Gestalt aufweist.

10. Sensor gemäß irgendeinem der Ansprüche 6 bis 9, verwendet zur Messung von Superoxidanionradikalen.

11. Verfahren zum Detektieren aktiver Sauerstoffspezies in einer Probe, umfassend das Messen eines Stroms, der durch eine Oxidations/Reduktions-Reaktion zwischen einem Metall in einer Metall-Porphyrin-Polymermembran und aktiven Sauerstoffspezies erzeugt wird, unter Verwendung des Sensors gemäß irgendeinem der Ansprüche 6 bis 10.

12. Verfahren gemäß Anspruch 11, wobei die zu detektierenden aktiven Sauerstoffspezies Superoxidanionradikale sind.

## Revendications

1. Electrode pour les espèces à oxygène actif comprenant un composant conducteur avec une membrane de polymère d'un complexe métal-porphyrine représenté par la formule (I) ou (II) suivante formée sur la surface, 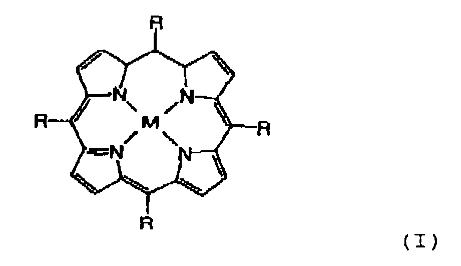 dans laquelle M est un métal choisi dans le groupe constitué du fer, du manganèse, du cobalt, du chrome et de l'iridium, au moins l'un des quatre R est un groupe choisi dans le groupe constitué d'un groupe thiofuryle, un groupe pyrtrolyle, un groupe furyle, un groupe mercaptophényle et un groupe aminophényle, et les autres R représentent l'un quelconque de ces groupes, un groupe alkyle, un groupe aryle ou un hydrogène, 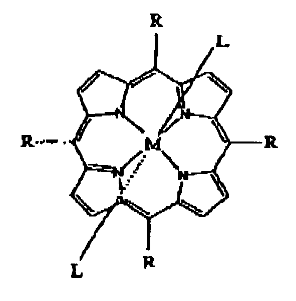 dans laquelle M et R sont comme définis ci-dessus, au moins l'un des deux L est un ligand axial contenant de l'azote tel que l'imidazole et ses dérivés, la pyridine et ses dérivés, l'aniline et ses dérivés, l'histidine et ses dérivés, et la triméthylamino et ses dérivés, un ligand axial contenant du soufre tel que le thiophénol et ses dérivée, la cystéine et ses dérivés, et la méthionine et ses dérivés, ou un ligand axial contenant de l'oxygène tel que l'acide benzoïque et ses dérivés, l'acide acétique et ses dérivés, le phénol et ses dérivés, un alcool aliphatique et ses dérivés, et l'eau, et l'autre L est l'un quelconque de ces ligands axiaux ou un groupe sans ligand.

2. Electrode selon la revendication 1, dans laquelle le composé de porphyrine formant le complexe métal porphyrine est choisi dans le groupe constitué des
5,10,15,20-tétrakis(2-thiofuryl)porphyrine,
5,10,15,20-térrakis(3-thiofuryl)porphyrine,
5,10,15,20-tétrakis(2-pyrrolyl)porphyrine,
5,10,15,20-tétrakis(3-pyrrolyl)porphyrine,
5,10,15,20-tétrakis(2-furyl)porphyrine,
5,10,15,20-tétrakis(3-furyl)porphyrine,
5,10,15,20-tétrakis(2-mercaptophényl)porphyrine,
5,10,15,20-tétrakis (3-mercaptophényl)porphyrine,
5,10,15,20-tétrakis(4-mercaptophényl)porphyrine,
5,10,15,20-tétrakis(2-aminophényl)porphyrine,
5,10,15,20-tétrakis(3-aminophényl)porphyrine,
5,10,15,20-tétrakis(4-aminophényl)porphyrine,
[5,10,15-tris(2-thiofuryl)-20-mono(phényl)]porphyrine,
[5,10,15-tris(3-thiofuryl)-20-mono(phényl)]porphyrine,
[5,10-bis(2-thiofuryl)-15,20-di(phényl)]porphyrine,
[5,10-bis(3-thiofuryl)-15,20-di(phényl)]porphyrine,
[5,15-bis(2-thiofuryl)-10,20-di(phényl)]porphyrine,
[5,15-bis(3-thiofuryl)-10,20-di(phényl)]porphyrine,
[5-mono(2-thiofuryl)-10,15,20-tri(phényl)]porphyrine, et
[5-mono(3-thiofuryl)-10,15,20-tri(phényl)]porphyrine,

3. Electrode selon la revendication 1 ou la revendication 2, dans laquelle le composant conducteur est inséré dans un petit tube préparé à partir d'un matériau électriquement isolant, l'extérieur de ce tube est recouvert d'un matériau agissant en tant que contre-électrode tel qu'un métal pour former une contre-électrode, une membrane de polymère de métal-porphyrine est formée sur l'extrémité du composant conducteur, et l'électrode a une forme d'aiguille.

4. Electrode selon la revendication 1 ou la revendication 2, dans laquelle le composant conducteur est inséré dans un matériau électriquement isolant, le matériau électriquement isolant est placé dans un matériau de contre-électrode, le matériau de contre-électrode résultant est logé dans un matériau électriquement isolant, dont l'extérieur est recouvert avec un matériau agissant en tant que masse, une membrane de polymère de métal-porphyrine est formée sur l'extrémité du composant conducteur, et l'électrode a une forme d'aiguille.

5. Electrode selon l'une quelconque des revendications précédentes, utilisée pour mesurer les radicaux d'anion superoxyde.

6. Capteur pour mesurer la concentration d'espèces à oxygène actif comprenant une électrode pour les espèces à oxygène actif comprenant un composant conducteur avec une membrane de polymère d'un complexe métal-porphyrine représenté par la formule (I) ou (II) suivante formée sur la surface, une contre-électrode, et une électrode de référence, 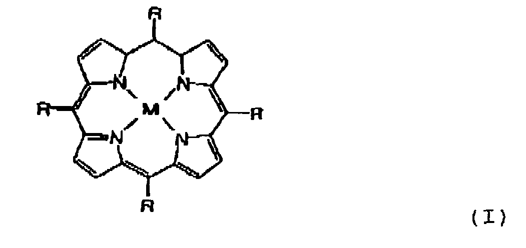 dans laquelle M est un métal choisi dans le groupe constitué du fer, du manganèse, du cobalt, du chrome et de l'iridium, au moins l'un des quatre R est un groupe choisi dans le groupe constitué d'un groupe thiofuryle, un groupe pyrrolyle, un groupe furyle, un groupe mercaptophényle et un groupe aminophényle, et les autres R représentent l'un quelconque de ces groupes, un groupe alkyle, un groupe aryle ou un hydrogène, 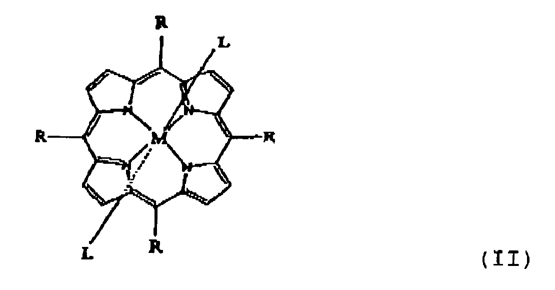 dans laquelle M et R sont comme définis ci-dessus, au moins l'un des deux L est un ligand axial contenant de l'azote tel que l'imidazole et ses dérivés, la pyridine et ses dérivés, l'aniline et ses dérivés, l'histidine et ses dérivés, et la triméthylamine et ses dérivés, un ligand axial contenant du soufre tel que le thiophénol et ses dérivés, la cystéine et ses dérivés, et la méthionine et ses dérivés, ou un ligand axial contenant de l'oxygène tel que l'acide benzoïque et ses dérivés, l'acide acétique et ses dérivés, le phénol et ses dérivés, un alcool aliphatique et ses dérivés, et l'eau, et l'autre L est l'un quelconque de ces ligands axiaux ou un groupe sans ligand.

7. Capteur selon la revendication 6, dans lequel le composé de porphyrine formant le complexe métal porphyrine est choisi dans le groupe constitué des
5,10,15,20-tétrakis(2-thiofuryl)porphyrine,
5,10,15,20-tétrakis(3-thiofuryl)porphyrine,
5,10,15,20-tétrakis(2-pyrrolyl)porphyrine,
5,10,15,20-tétrakis(3-pyrrolyl)porphyrine,
5,10,15,20-tétrakis(2-furyl)porphyrine,
5,10,15,20-tétrakis(3-furyl)porphyrine,
5,10,15,20-tétrakis(2-mercaptophényl)porphyrine,
5,10,15,20-tétrakis(3-mercaptophényl)porphyrine,
5,10,15,20-tétrakis(4-mercaptophényl)porphyryrine,
5,10,15,20-tétrakis(2-aminophényl)porphyrine,
5,10,15,20-tétrakis(3-aminophényl)porphyrine,
5,10,15,20-tétrakis(4-aminophényl)porphyrine,
[5,10,15-tris(2-thiofuryl)-20-mono(phényl)]porphyrine,
[5,10,15-tris(3-thiofuryl)-20-mono(phényl)]porphyrine,
[5,10-bis(2-thiofuryl)-15,20-di(phényl)]porphyrine,
[5,10-bis(3-thiofuryl)-15,20-di(phényl)]porphyrine,
[5,15-bis(2-thiofuryl)-10,20-di(phényl)]porphyrine,
[5,15-bis(3-thiofuryl)-10,20-di(phényl)]porphyrine,
[5-mono(2-thiofuryl)-10,15,20-tri(phényl)]porphyrine, et
[5-mono(3-thiofuryl)-10,15,20-tri(phényl)]porphyrine.

8. Capteur selon la revendication 6 ou la revendication 7, dans lequel une électrode est utilisée, l'électrode comprenant, un composant conducteur inséré dans un petit tube préparé à partir d'un matériau électriquement isolant, l'extérieur de ce tube étant recouvert d'un matériau agissant en tant que contre-électrode tel qu'un métal pour former une contre-électrode, une membrane de polymère de métal-porphyrine étant formée sur l'extrémité du composant conducteur, et l'électrode ayant une forme d'aiguille.

9. Capteur selon la revendication 6 ou la revendication 7, dans lequel une électrode est utilisée, l'électrode comprenant un composant conducteur inséré dans un matériau électriquement isolant, le matériau électriquement isolant étant placé dans un matériau de contre-électrode, le matériau de contre-électrode résultant étant logé dans un matériau électriquement isolant, dont l'extérieur est recouvert avec un matériau agissant en tant que masse, une membrane de polymère de métal-porphyrine étant formée sur l'extrémité du composant conducteur, et l'électrode ayant une forme d'aiguille.

10. Capteur selon l'une quelconque des revendications 6 à 9, utilisé pour mesurer les radicaux d'anion superoxyde.

11. Procédé de détection des espèces à oxygène actif dans un échantillon comprenant la mesure d'un courant produit par une réaction d'oxydoréduction entre un métal dans une membrane de polymère de métal-porphyrine et une espèce à oxygène actif en utilisant le capteur selon l'une quelconque des revendications 6 à 10.

12. Procédé selon la revendication 11, dans lequel les espèces à oxygène actif sont des radicaux d'anion superoxide.
